# EUROPÄISCHE PATENTSCHRIFT

(11) **EP 0 995 099 B1**
(45) Veröffentlichungstag und Bekanntmachung des Hinweises auf die Patenterteilung: **07.05.2003**
(21) Anmeldenummer: 98940195.5
(22) Anmeldetag: 10.07.1998
(51) Int. Cl.: G01N 7/14, G01N 33/44, B29C 44/60

(54) **MESSEINRICHTUNG UND VERFAHREN ZUR MESSUNG DER GASBELADUNG VON FLÜSSIGEN KUNSTSTOFFKOMPONENTEN**
MEASURING DEVICE AND METHOD FOR MEASURING GAS LOAD IN LIQUID PLASTIC MATERIALS
DISPOSITIF DE MESURE ET PROCEDE POUR MESURER LA CHARGE DE GAZ DE COMPOSANTS LIQUIDES DE MATIERES PLASTIQUES

(30) Priorität: 11.07.1997 DE 29712263 U
(43) Veröffentlichungstag der Anmeldung: 26.04.2000
(73) Patentinhaber: EDF Polymer-Applikation Maschinenfabrik GmbH, 6912 Hörbranz (AT)
(72) Erfinder: FETZ, Dietmar, A-6890 Lustenau (AT); HAUSBICHLER, Hannes, A-6900 Bregenz (AT)
(74) Vertreter: Hennicke, Ernst Rüdiger, Dipl.-Ing.
(86) Internationale Anmeldenummer: EP9804280
(87) Internationale Veröffentlichungsnummer: WO99002963

(56) Entgegenhaltungen:
- EP-A- 0 516 904
- WO-A-92/08121
- DE-A- 3 830 209
- US-A- 3 673 853
- US-A- 3 853 500
- US-A- 4 299 794
- US-A- 4 376 172
- US-A- 4 862 729
- PATENT ABSTRACTS OF JAPAN vol. 010, no. 051 (M-457), 28. Februar 1986 & JP 60 201918 A (NIIGATA TEKKOSHO KK;OTHERS: 01), 12. Oktober 1985

## Beschreibung

### Technisches Gebiet

Die Erfindung betrifft eine Meßeinrichtung zur Messung der Gasbeladung einer flüssigen Kunststoffkomponente, entsprechend der im Oberbegriff des Anspruchs 1 angegebenen Art. Die Erfindung ist ferner auf ein Verfahren zur Bestimmung der Gasbeladung einer Flüssigkeit insbesondere unter Verwendung der erfindungsgemäßen Meßeinrichtung gerichtet.

### Stand der Technik

In der Technik, vor allem in der Kunststoffverarbeitungstechnik, ist es häufig erforderlich, die Gasbeladung von Flüssigkeiten, z.B. die Luftbeladung flüssiger Kunststoffkomponenten für die Schaumstofferzeugung zu ermitteln, zumeist mit dem Ziel, im laufenden Arbeitsbetrieb mit einer konstanten Gasbeladung arbeiten zu können.

Für die Bestimmung der Gasbeladung von Flüssigkeiten und vor allem von flüssigen Kunststoffkomponenten sind seit langem zahlreiche Meßeinrichtungen bekannt (DE 37 20 904 A1, DE 33 36 037 A1, DE 31 32 597 C2, DE 38 30 209 A1, DE 41 19 966 A1, EP 0 125 541 A2). Die bekannten Meßeinrichtungen verwenden im allgemeinen mit Meßkolben versehene Meßzylinder, in die aus dem die gasbeladene Flüssigkeit führenden System in Intervallen eine bestimmte Probemenge abgezogen wird, die dann im Meßzylinder gegenüber dem System isoliert vermessen wird. Dies erfolgt im allgemeinen in der Weise, daß die mit dem gelösten und ggf. auch freiem Gas beladene Probemenge im geschlossenen Meßzylinderraum unter Freisetzung des Gases einem Unterdruck ausgesetzt wird, indem das Volumen des Meßzylinderraums durch entsprechende Kolbenverstellung erhöht wird. Aus den ermittelten Volumenveränderungen und den sich ändernden Drücken der Meßprobe läßt sich die Gasbeladung der Flüssigkeit nach den bekannnten physikalischen Beziehungen (Gasgesetz) errechnen. Gleiches ist möglich, wenn die Meßprobe im Meßzylinder durch Kompression mittels des Meßkolbens und/oder durch eine Kombination von Dekompression und anschließender Kompression mit Druckmessung unter den verschiedenen Prüfbedingungen und gleichzeitiger Volumenbestimmung des Meßraumes bei den verschiedenen Kolbenstellungen vermessen wird.

Aus der US-PS 4 376 172 ist eine Meßeinrichtung nach dem Oberbegriff des Anspruches 1 bekannt, deren Meßzylinderraum eingangsseitig über ein Füllventil an das die gasbeladene Flüssigkeit unter Druck führende, einen Vorratsbehälter für die gasbeladene Flüssigkeit aufweisende System und ausgangsseitig über ein Ausstoßventil an eine zu dem Vorratsbehälter zurückführende Rücklaufleitung angeschlossen ist. Auch hier erfolgt die Messung der Gasbeladung dadurch, daß dem System eine bestimmte Probemenge entzogen und dem Meßzylinder der Meßpumpe zugeführt wird und dann bei geschlossenen Füll- und Ausstoßventilen, also von dem System isoliert vermessen wird, wozu durch weggesteuerte Kolbenverstellung zunächst ein Unterdruck und dann ein Überdruck im Meßzylinder erzeugt wird. Die gemessenen Druckwerte sowie die sich aus den Hubbewegungen des Meßkolbens ergebenden Volumenveränderungen des Meßzylinderraumes werden dabei von einer elektronischen Auswerteeinheit verarbeitet und zur Steuerung der Gasbeladung der Flüssigkeit auf einen vorgegebenen Konstantwert genutzt . Nach erfolgtem Meßvorgang werden das Füllventil und das Auslaßventil wieder geöffnet, so daß die Meßprobe durch den Pumpendruck im System wieder in den Vorratsbehälter zurückgeführt wird. In den Intervallen zwischen den periodischen Probemessungen ist der Pumpen-Meßzylinder ständig an das Pumpensystem der Gesamtanlage angeschlossen.

### Darstellung der Erfindung

Die Erfindung geht aus von den bekannten Meßeinrichtungen, insbesondere der mit einer Meßpumpe arbeitenden Einrichtung nach der vorgenannten US-PS 4 376 172.

Aus der US 4 299 794 ist ein medizinisches Meßgerät bekannt, mit welchem in einem Blutserum oder Urin der Gehalt von gelöstem oder chemisch gebundenem CO₂ gemessen werden kann. Das Meßgerät umfaßt einen Zylinder als Probenraum, einen beweglichen Kolben, ein Einlaßventil und einen Meßumformer. Bevor die Meßprobe vermessen wird, wird ihr eine Säure zugeführt, durch die sich die Zusammensetzung der Meßprobe ändert. Nach der Messung muß die Meßprobe daher entsorgt werden.

Aus der US 3 853 500 ist eine Entgasungsvorrichtung bekannt, mit der in einer Flüssigkeit enthaltene Gasblasen aus dieser entfernt werden können. Zur Beschleunigung der Entgasung wird die Flüssigkeit einem Ultraschallfeld ausgesetzt.

Aus der EP 0 516 904 A1 sind eine Vorrichtung und ein Verfahren bekannt, mit denen Treibmittel in eine flüssige Kunststoffkomponente eingebunden werden können. In einer Meßeinrichtung kann anschließend das Volumenausdehnungsvermögen des Material-Treibmittelgemisches bestimmt werden, wobei während der Expansion der Probe der Druck, das Volumen und die Temperatur der Probe gemessen werden, um anhand der idealen Gasgleichung den molaren Anteil des Treibmittels zu bestimmen.

Aus der EP 525 933 A2 ist eine Vorrichtung bekannt, mit welcher die Zustände in Hochleistungstransformatoren, wie sie beispielsweise bei Lokomotiven zum Einsatz kommen, bestimmt werden können. Hierbei umfaßt die Vorrichtung eine Einrichtung, mit welcher das Volumen und die Zusammensetzung der im Kühlöl für den Transformator eingebundenen oder gelösten Gase bestimmt werden kann. Die Vorrichtung verwendet hierzu zwei separat voneinander arbeitende Kolbenpumpen.

Die US 4 090 695 betrifft die Verwendung von Kolbenpumpen bei der Speisung von Mischkammern mit flüssigen Kunststoffkomponenten für den Spritzguß. Der Pumpkolben der Kolbenpumpe kann jeweils auf dieselbe Startposition zurückgesetzt werden, so daß durch einen unterschiedlichen Kolbenhub das Füllvolumen für die einzelnen Mischkammern bestimmt werden kann.

Aufgabe der Erfindung ist es vor allem, daß aus der US 4 376 172 bekannte System, insbesondere die hierbei als Meßeinrichtung für die Gasbeladung verwendete Kolbenpumpe, ohne übermäßigen Bauaufwand so auszugestalten, daß eine genaue Messung der Gasbeladung von flüssigen Kunststoffkomponenten für die Schaumstofferzeugung, in verschiedenen Arbeitssystemen und bei unterschiedlichen Anordnungen der Kolbenpumpe, erreichbar ist.

Diese Aufgabe wird hinsichtlich ihres vorrichtungsmäßigen Aspektes durch die in Patentanspruch 1 und hinsichtlich ihres verfahrensmäßigen Aspektes durch die in Patentanspruch 24 angegebene Erfindung gelöst.

Bei der erfindungsgemäßen Ausgestaltung der Meßeinrichtung ist mit Hilfe des gesteuerten Stellmotors und des zugeordneten Übertragungsgetriebes eine sehr genaue Hubverstellung des Pumpenkolbens in Saugrichtung wie auch in Druckrichtung möglich und folglich eine genaue Bestimmung der sich beim Meßvorgang einstellenden Volumenveränderung des Meßzylinderraumes, so daß über die hubabhängigen Volumenveränderungen und die dabei gemessenen Druckwerte der isolierten Meßprobe der Gasgehalt der flüssigen Meßprobe exakt bestimmt werden kann. Zugleich kann die erfindungsgemäße Kolbenpumpe als Förderpumpe arbeiten, die nach erfolgter Messung die Meßprobe bei geöffnetem Ausstoßventil aus dem den Meßzylinderraum bildenden Pumpenzylinder herausdrückt. Damit ergibt sich auch die Möglichkeit, die erfindungsgemäße Kolbenpumpe auch in einem eigenen, vom Pumpensystem der Gesamtanlage getrennten Umlaufkreis anzuordnen und den Meßzylinderraum der Kolbenpumpe in den Intervallen zwischen den Meßvorgängen gegenüber dem druckführenden System abzusperren. Vorzugsweise werden hierbei für das der Kolbenpumpe zugeordnete Füllventil und das Ausstoßventil unabhängig voneinander von der Schließstellung in die Öffnungsstellung und umgekehrt schaltbare Ventile verwendet, so daß eine Befüllung des Meßzylinderraums mit der Meßprobe bei geöffnetem Füllventil und geschlossenem Ausstoßventil und nach erfolgtem Meßvorgang ein Ausstoßen der Meßprobe bei geschlossenem Füllventil und geöffnetem Ausstoßventil möglich ist.

In weiterer vorteilhafter Ausgestaltung der Erfindung ist die der Kolbenpumpe zugeordnete Meßvorrichtung so ausgebildet, daß sie sowohl die Drücke der Meßprobe bei den unterschiedlichen Volumina des Meßzylinderraumes wie auch die Temperatur der Meßprobe mißt. Damit läßt sich auch der Einfluß der Temperatur auf das Gasabsorptionsvermögen der Flüssigkeit meßtechnisch erfassen und z.B. zur Erzielung einer konstanten Gasbeladung der Flüssigkeit im Betrieb auswerten.

Für den Stellantrieb wie auch für das Übertragungsgetriebe lassen sich bei der erfindungsgemäßen Kolbenpumpe unterschiedliche Antriebsteile verwenden. Für den Stellmotor kann mit Vorteil ein in seiner Drehrichtung reversierbarer, vorzugsweise elektrischer, Drehmotor nach Art eines Schrittmotors verwendet werden. Eine vorteilhafte Ausführungsform des Übertragungsgetriebes besteht aus einem Spindelgetriebe, mit dessen gegen Drehung gesicherter axial verstellbarer Spindelmutter der Pumpenkolben gekoppelt wird. Die erfindungsgemäß vorgesehene Kolbenpumpe läßt sich als Kompaktgerät ausführen. Sie weist zweckmäßig einen den Meßzylinderraum aufweisenden Zylinderkörper auf, der als Grundkörper über einen das Übertragungsgetriebe bzw. das Spindelgetriebe aufnehmenden, vorzugsweise zylindrischen, Gehäusekörper mit einem Anschlußkörper für den Anbau des Stellmotors verbunden wird. Dabei können die verschiedenen Bauteile zweckmäßig durch Verschraubung od.dgl. lösbar miteinander verbunden sein. Der genannte Anschlußkörper, an dem sich der Stellmotor anflanschen läßt, kann ein Gehäuse für die Aufnahme einer den Stellmotor mit dem Übertragungsgetriebe bzw. dem Spindelgetriebe kuppelnden Kupplungsvorrichtung bilden.

Eine ganz besonders vorteilhafte Ausgestaltung der Erfindung, der in Verbindung mit dem Oberbegriff des Anspruches 1 selbständig schutzfähige Bedeutung zukommt, ergibt sich, wenn dem Meßzylinderraum der Meßeinrichtung ein Ultraschallerzeuger zugeordnet wird. Mit Hilfe des Unterschallerzeugers können in der im Meßzylinderraum aufgenommenen Flüssigkeitsprobe Ultraschallschwingungen erzeugt werden, während der Pumpenkolben zur Verringerung des Druckes im Meßraum verfahren oder auch auf Meßdruck gehalten wird. Die Ultraschallschwingungen haben zur Folge, daß das in der Flüssigkeit enthaltene Gas bedeutend schneller ausgetrieben wird, als dies allein durch den erzeugten Unterdruck möglich ist. Durch den Unterdruckerzeuger in Verbindung mit dem Ultraschallerzeuger kann damit die Zeit, die zum genauen Vermessen einer Probe erforderlich ist, erheblich verkürzt werden. In Einzelfällen verringert sich die Zeit vom Einbringen der Probemenge in den Meßzylinder bis zu dem Moment, in dem ein verwertbares Meßergebnis vorliegt, durch die Erzeugung von Ultraschallwellen in der Probe auf ein Viertel gegenüber der Zeit, die für eine verläßliche Messung ohne Ultraschall erforderlich ist. Das erfindungsgemäße Verfahren erlaubt somit nicht nur eine besonders genaue, sondern auch sehr zügige Bestimmung der Gasbeladung.

Zweckmäßig münden der Einlaß und der Auslaß für die Befüllung und Entleerung des Meßzylinderraums radial seitlich in diesen. Der Ultraschallerzeuger kann dann an einem axialen Ende des Meßzylinderraums angeordnet sein, wodurch sich eine besonders kompakte Anordnung und eine leichte Zugänglichkeit zum Ultraschallerzeuger ergibt. Vorzugsweise ist dieser auswechselbar in einer Öffnung im Meßzylinderraum aufgenommen, wobei es zur Vermeidung eines direkten Kontaktes zwischen der zu vermessenden Flüssigkeit und dem Ultraschallerzeuger vorteilhaft sein kann, wenn die Öffnung von einer Membran verschlossen ist. In diesem Fall muß jedoch sichergestellt sein, daß die vom Ultraschallerzeuger erzeugten Schwingungen von der Membran unbehindert in die Flüssigkeit eingeleitet werden. Eine besonders günstige Lösung ergibt sich, wenn der Ultraschallerzeuger in einem Halteflansch aufgenommen und an diesem mittels Befestigungsschrauben gesichert ist, so daß er leicht ausgetauscht werden kann, wenn dies beispielsweise im Falle einer Funktionsstörung erforderlich sein sollte.

Es ist auch denkbar, anstelle des Ultraschallwellenerzeugers einen anderen Schwingungserzeuger vorzusehen, beispielsweise einen rotierenden Exzenter o.dgl., der an der Kolbenpumpe Schwingungen niedrigerer Frequenz erzeugt, die in die Flüssigkeitsprobe übertragen werden und so ein schnelleres Austreiben des Gases aus der Flüssigkeit bewirken.

Der Meßzylinderraum kann an seiner Zylinderwand bzw. dem Zylinderkörper mindestens eine Anschlußöffnung für einen Druckund/oder Temperaturfühler aufweisen, womit die Drücke und Temperaturen in den einzelnen Meßzuständen ermittelt werden können. In der Zylinderwand zum Meßzylinderraum kann eine verschließbare Kontroll- und/oder Serviceöffnung vorgesehen sein, um durch ein Sichtfenster od.dgl. Einblick in den Meßzylinderraum zu gewähren oder beispielsweise zur Reinigung einen Zugang zu schaffen, ohne daß die gesamte Apparatur vollständig auseinandergenommen werden muß.

Mit besonderem Vorteil läßt sich die erfindungsgemäße Meßeinrichtung in einem System, insbesondere einem Kunststoff-Verarbeitungssystem verwenden, wobei der Meßzylinderraum der Kolbenpumpe eingangsseitig über das Füllventil möglichst unmittelbar, in jedem Fall zweckmäßig mit einer möglichst kurzen Leitung an einen die gasbeladene Flüssigkeit unter einem Vordruck enthaltenden, mit einer Gaszuführung versehenen Behälter und ausgangsseitig über das Ausstoßventil an den Rücklauf zum Vorratsbehälter angeschlossen ist. Der Umlaufkreis für die Probemessungen kann gegenüber dem aus dem Vorratsbehälter mit der gasbeladenen Flüssigkeit versorgten System über ein Absperrventil absperrbar sein, so daß der Systemdruck eingangsseitig nicht an der Kolbenpumpe ansteht. Im übrigen weist das System, wie an sich bekannt, zweckmäßig eine Auswerte- und Regeleinrichtung auf, die über elektrische Signal- und Steuerleitungen mit der den Druck und zweckmäßig auch die Temperatur der Meßprobe messenden Meßvorrichtung und mit dem Stellantrieb sowie zweckmäßig auch einem Gaszuführungsventil verbunden ist, das in einer zu dem Vor-ratsbehälter führenden Gaszuführungsleitung angeordnet ist und mit dessen Hilfe die Gasbeladung der im Vorratsbehälter befindlichen Flüssigkeit auf einem konstanten Sollwert eingestellt wird.

Weitere Merkmale und Vorteile der Erfindung ergeben sich aus den einzelnen Unteransprüchen und der nachfolgenden Beschreibung der in der Zeichnung gezeigten, bevorzugten Ausführungsbeispiele. Hierbei zeigen:

### Kurze Beschreibung der Zeichnungen

- Fig. 1: eine erfindungsgemäße Gesamteinrichtung zusammen mit der zur Messung der Gasbeladung der Flüssigkeit verwendeten, hier nur schematisch wiedergegebenen Kolbenpumpe;
- Fig. 2: eine erste Ausführungsform der in Fig. 1 gezeigten Kolbenpumpe in einem Axialschnitt;
- Fig. 3: einen Querschnitt nach Linie III-III der Fig. 2;
- Fig. 4: einen Querschnitt nach Linie IV-V der Fig. 2.
- Fig. 5: eine zweite, bevorzugte Ausführungsform der Kolbenpumpe in einer Fig. 2 entsprechenden, jedoch lediglich in einem Ausschnitt vergrößert wiederge-gebenen Darstellung.

### Bevorzugte Wege zur Ausführung der Erfindung

Zum Verständnis der Erfindung wird Bezug genommen auf die eingangs zum Stand der Technik genannten Druckschriften, deren Offenbarungsinhalt zum Inhalt der vorliegenden Erfindungsbeschreibung gemacht wird.

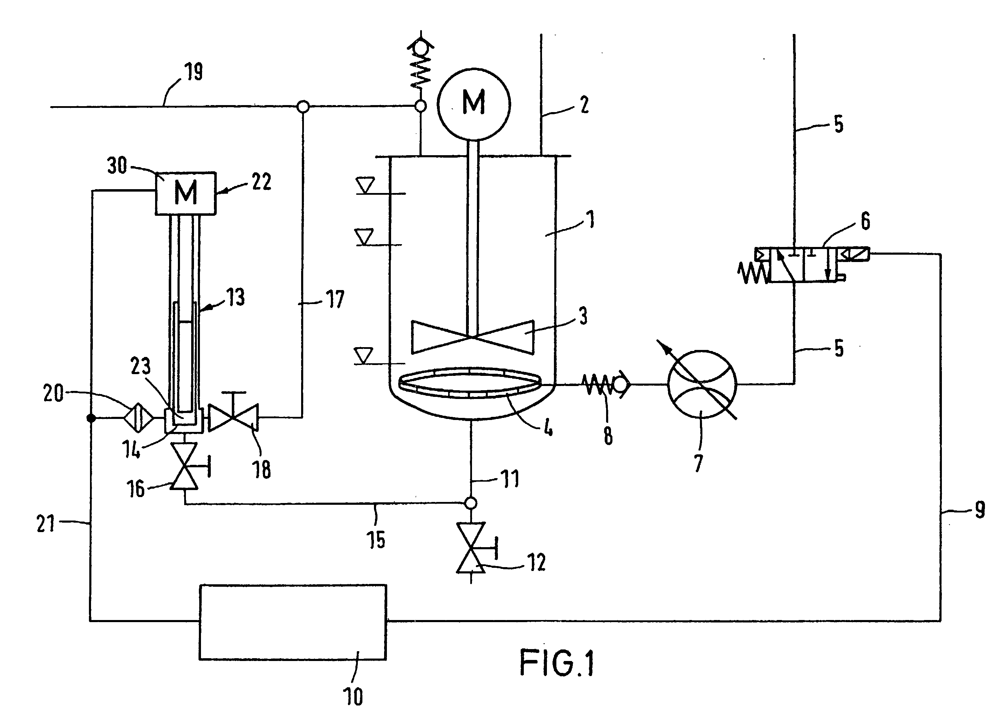

Das in Fig. 1 als Fließschema gezeigte Arbeitssystem ist Bestandteil einer Gesamtanlage, die bevorzugt für die Kunststoffverarbeitung und hier vor allem für die Erzeugung von Polyurethan-Schaumkunststoffen verwendbar ist. Das System weist einen Vorratsbehälter 1 auf, der eine gasbeladene Flüssigkeit (Kunststoffkomponente) aufnimmt, die über eine Leitung 2 dem Vorratsbehälter zugeführt und in diesem unter einem vorgegebenen, konstant gehaltenen Vordruck gehalten wird. Im Vorratsbehälter 1 befindet sich, wie an sich bekannt, ein von einem Motor angetriebenes Rührwerk 3 und im Bereich darunter ein ringförmiger Verteiler 4, über die ein Gas, z.B. Luft, in den Vorratsbehälter 1 eingeleitet und in der darin befindlichen Flüssigkeit fein verteilt wird, so daß es zumindest überwiegend von der Flüssigkeit absorbiert wird. Der Verteiler 4 ist über eine Leitung 5 an eine geeignete Gasquelle für die Zuleitung des der Gasbeladung der Flüssigkeit dienenden Gases angeschlossen, wobei in der Leitung 5 ein Gaszuführungsventil 6, hinter diesem ein Durchflußmengenregler 7 sowie ein Rückschlagventil 8 angeordnet sind. Das Gaszuführungsventil 6 ist als Magnetventil ausgebildet, das über eine elektrische Steuerleitung 9 von einer zentralen elektrischen Auswerte- und Steuereinheit 10 in Öffnungsrichtung angesteuert werden kann, um die Verbindung der Leitung 5 zu dem Verteiler 4 zur Einführung des Gases in die im Vorratsbehälter 1 befindliche Flüssigkeit herzustellen.

Der Vorratsbehälter 1 ist an seinem Boden an eine Auslaßleitung 11 angeschlossen, die bei der Anlage für die Kunststoffverarbeitung zu einem nicht-dargestellten Mischkopf führt, in dem die gasbeladene Komponente aus dem Vorratsbehälter 1 mit einer zweiten Kunststoffkomponente gemischt wird, bevor das Reaktionsgemisch zur Ausschäumung gebracht wird. In der Auslaßleitung 11 befindet sich ein Absperrventil 12.

Um die Gasbeladung der im Vorratsbehälter 1 befindlichen Flüssigkeit auf einen vorgegebenen Sollwert einzustellen und auf diesem Sollwert zu halten, ist eine Meßeinrichtung zur Messung der Gasbeladung der Flüssigkeit vorgesehen, die als Meßgerät eine Kolbenpumpe 13 aufweist, deren Pumpenzylinder einen Meßzylinderraum 14 zur Aufnahme einer Probemenge der aus dem Vorratsbehälter 1 entnommenen gasbeladenen Flüssigkeit bildet. Dabei ist der Meßzylinderraum 14 über eine Zuführungsleitung 15, in der sich ein Füllventil 16 befindet, an den Vorratsbehälter 1 bzw. im gezeigten Ausführungsbeispiel an die Auslaßleitung 11 angeschlossen, und zwar in Strömungsrichtung vor dem Absperrventil 12. Es versteht sich, daß die Zufuhrleitung 15 einschließlich des Ventils 16 möglichst kurz gehalten ist, so daß die Probe zur Vermessung auch tatsächlich frisch aus dem Behälter 1 entnommen wird und nicht vorher längere Zeit in der Leitung 15 selbst abgestanden ist.

Der Meßzylinderraum 14 der Kolbenpumpe 13 ist weiter über eine Rückführleitung 17, in der sich ein Ausstoßventil 18 befindet, an den Vorratsbehälter 1 angeschlossen, wobei hier in dem dargestellten Beispiel die Rückführleitung in einen Rücklauf 19 mündet, der eine Rücklaufverbindung für überschüssige, unvermischte Flüssigkeit von dem vorgenannten Mischkopf zu dem Vorratsbehälter 1 bildet. Es ist ersichtlich, daß die Kolbenpumpe 13 in einem von den Leitungen 15 und 17 gebildeten Umlaufkreis angeordnet ist, in den der Vorratsbehälter 1 eingeschaltet ist und der bei geschlossenem Absperrventil 12 von dem Systemdruck in der Anlage abgesperrt ist. Möglich ist selbstverständlich auch eine Anordnung, bei der die den Umlaufkreis bildenden Leitungen 15 und 17, statt an die Leitungen 11 und 19, unmittelbar an den Vorratsbehälter 1 angeschlossen sind, so daß auch in diesem Fall ein vom Systemdruck unabhängiger Meßproben-Umlaufkreis gebildet wird.

Dem Meßzylinderraum 14 der Kolbenpumpe 13 ist gem. Fig. 1 eine Meßvorrichtung 20 zugeordnet, die die unterschiedlichen Drücke der als Meßprobe im Meßzylinderraum 14 befindlichen gasbeladenen Probemenge bei den verschiedenen Kolbenstellungen der Kolbenpumpe mißt und die vorzugsweise auch die Temperatur der Meßprobe während der Meßphase mißt, wobei die ermittelten Druck- und Temperaturmeßwerte über eine elektrische Signalleitung 21 der Auswerte- und Steuereinheit 10 zur Auswertung zugeführt werden. Die elektrische Signalleitung 21 dient ferner zur Steuerung des Stellantriebs 22 der Kolbenpumpe 13.

Zur Messung der Gasbeladung der im Vorratsbehälter 1 befindlichen Flüssigkeit wird diese am Behälterboden vor dem Absperrventil 12 über die Leitungen 11, 15 abgezogen und über das geöffnete Füllventil 16 in den Meßzylinderraum 14 der Kolbenpumpe 13 eingeführt. Hierbei führt der den Meßkolben bildende Pumpenkolben 23 einen Saughub aus, im gezeigten Ausführungsbeispiel also einen Aufwärtshub, wobei das Volumen des Meßzylinderraumes 14 sich entsprechend vergrößert. Sobald der Pumpenkolben 23 eine erste obere Endlage im Meßzylinderraum 14 erreicht hat, bei der die gewünschte Probemenge im Meßzylinderraum enthalten ist, wird das Füllventil 16 geschlossen, so daß die in den Meßzylinderraum 14 eingezogene Probemenge bei gleichfalls geschlossenem Ausstoßventil 18 im Meßzylinderraum zur Durchführung des Meßvorgangs eingeschlossen bzw. isoliert ist.

Anschließend wird die Gasbeladung der Probe bestimmt, wozu zunächst bei Stillstand des Pumpenkolbens 23 und bei geschlossenen Ventilen 16 und 18 deren Druck und die Temperatur der Meßprobe unmittelbar nach Einbringen in die Meßkammer 14 mit Hilfe der Meßvorrichtung 20 gemessen wird. Die dabei ermittelten Meßwerte werden über die Signalleitung 21 der Auswerte- und Steuereinheit 10 zugeführt. Anschließend wird der Pumpenkolben 23 um einen Hubweg weiter in Rückhubrichtung (Saugrich-tung) bewegt und dort gehalten, so daß die im Meßzylinderraum 14 eingeschlossene Meßprobe einem Unterdruck ausgesetzt wird, um das Trennen von Gas und Flüssigkeit, also das Exsorbieren des Gases aus der Flüssigkeit der Meßprobe zu erzwingen. Danach kann dann der Pumpenkolben 23 um einen vorbestimmten Hub zurückgefah-ren, d.h. in Druckrichtung bzw. im Sinne einer Verkleinerung des Volumens des Meßzylinderraumes 14 verstellt werden, beispielsweise derart, daß im Meßzylinderraum 14 ein vorbestimmter Referenzdruck (Prüfdruck), z.B. Atmosphärendruck in der Meßprobe er-reicht wird, der durch eine erneute Druck- und Temperaturmessung mit Hilfe der Meßvorrichtung 20 festgestellt wird, deren Meßsignale ebenfalls über die Signalleitung 21 der Auswerte- und Steuereinheit 10 zugeführt werden. Die Auswerte- und Steuereinheit 10 kann nun unter Zugrundelegung des Gasgesetzes (p x V = m x R x T) über die ihr zugeführten Druck- und Temperaturmeßwerte und die Hubwege des Pumpenkolbens 23, aus welchen sich die Volumenveränderungen des Meßzylinderraumes 14 ergeben, die in der Meßprobe ursprünglich enthaltene Gasmenge berechnen. Weicht diese Gasmenge von einem vorgegebenen Sollwert ab, so kann über die Auswerte- und Steuereinheit 10 und das von ihr angesteuerte Gaszuführungsventil 6 die Gasmenge der im Vorrats-behälter 1 befindlichen Flüssigkeit durch Zuführung oder Ableitung von Gas auf den Sollwert eingestellt werden. Nach erfolgtem Meßvorgang wird der Pumpenkolben 23 von dem Stellantrieb 22 in Druckrichtung (hier: in Abwärtsrichtung) angetrieben, so daß er die im Meßzylinderraum 14 befindliche Probemenge über das nun geöffnete Ausstoßventil 18 und die Rücklaufleitung 17 in den Vorratsbehälter 1 zurückführt. Es ist ersichtlich, daß die Messung der Gasbeladung der Flüssigkeit in einem Zirkulations-betrieb erfolgt, der durch aufeinanderfolgende Meßvorgänge solange durchgeführt wird, bis die dem Vorratsbehälter 1 entnom-mene Probemenge im Meßzylinderraum 14 der Kolbenpumpe 13 die-selbe Kondition (Gasbeladung) aufweist, wie sie durch den Soll-wert vorgegeben ist.

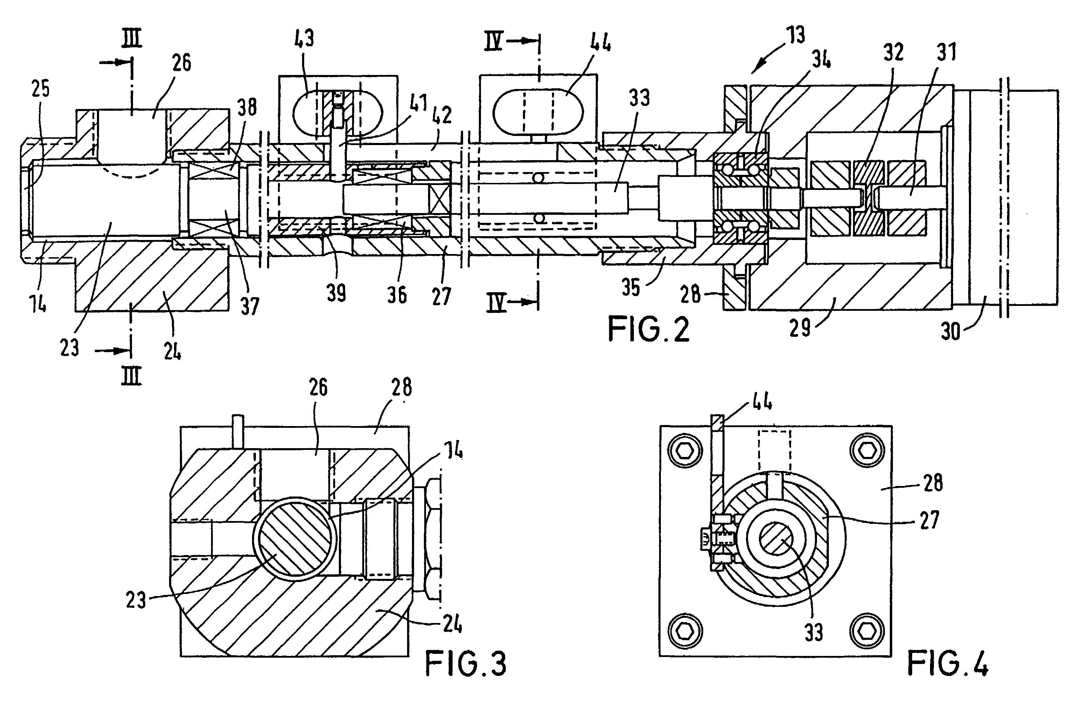

Im folgenden wird im Zusammenhang mit den Fig. 2 bis 5 der Aufbau der Kolbenpumpe 13 näher erläutert. Wie ersichtlich, weist die in den Fig. 2-4 gezeigte Ausführungsform der Kolbenpumpe 13 einen den Meßzylinderraum 14 als Zylinderbohrung aufweisenden, aus einem Metallblock bestehenden Zylinderkörper 24 auf, in dem der Pumpenkolben 23 mit Kolbendichtung geführt ist, wobei der Meßzylinderraum 14 endseitig den Einlaß 25 für die über die Zuführungsleitung 15 (Fig. 1) und das Füllventil 16 zuzuführende Probemenge und rechtwinklig hierzu den Auslaß 26 für den Anschluß der Rückführungsleitung 17 mit dem hier befindlichen Ausstoßventil 18 aufweist. Mit dem Zylinderkörper 24 ist auf der dem Einlaß 25 gegenüberliegenden Seite ein zylindrischer Gehäusekörper 27 verschraubt, der ein mit dem Kolben 23 verbundenes Übertragungsgetriebe in Gestalt eines Spindeltriebes aufnimmt. An seinem anderen Gehäuseende ist der Gehäusekörper 27 mit einer anschraubbaren Flanschplatte 28 lösbar an einem Anschlußkörper 29 angeschlossen, an dessen freiem Stirnende ein Stellmotor 30 des Stellantriebs 22 lösbar angebaut ist. Die Motorwelle 31 des Stellantriebs 22 ist über eine Kupplung 32 im Inneren des Anschlußkörpers 29 mit dem Übertragungsgetriebe bzw. dessen Hubspindel 33 antriebsmäßig gekoppelt, wobei die Kupplung 32 hier nach Art einer Steckkupplung ausgeführt ist. Die Hubspindel 33 ist mit einem Wälzlager 34 am Anschlußkörper 29 drehbar gelagert, und zwar in einer mittels der Flanschplatte 28 festgelegten Lagerhülse 35, mit der der zylindrische Gehäusekörper 27 endseitig verschraubt ist. Der Stellmotor besteht aus einem in seiner Drehrichtung reversierbaren elektrischen Schrittmotor, der die Hubspindel 33 in beiden Drehrichtungen anzutreiben vermag, um den Pumpenkolben 23 in beiden Hubrichtungen zu bewegen. Auf dem Außengewinde der Hubspindel 33 sitzt im Inneren des Gehäusekörpers 27 eine Spindelmutter 36, mit der der Pumpenkolben 23 antriebsmäßig gekoppelt ist.

Wie Fig. 2 zeigt, weist der Kolben 23 einen im Durchmesser abgesetzten Kolbenschaft 37 auf, an dem er mit einem Gleitlager 38 versehen und mit diesem axial verschieblich im zylindrischen Gehäusekörper 27 geführt ist. An den Kolbenschaft schließt sich ein Hohlkolbenansatz 39 an, der sich ebenfalls im Gehäusekörper 27 axial führt und in den die Hubspindel 33 mit ihrem kolbenseitigen Ende eintaucht, wobei die Spindelmutter 36 in dem Hohlkolbenansatz 39 angeordnet und mit diesem verbünden ist. An dem Hohlkolbenansatz 39 ist radial zu diesem eine Drehsicherung in Gestalt eines radial vorragenden Stiftes 41 fest angeordnet, der auch aus einer eingeschraubten Stiftschraube bestehen kann und eine axiale Schlitzöffnung 42 des Gehäusekörpers 27 durchfäßt, so daß bei angetriebener Hubspindel 33 der Pumpenkolben 23 und die mit ihm verbundene Spindelmutter 36 gegen Drehung gesichert sind, die Spindelmutter 36 mit dem Pumpenkolben 23 also nur eine axiale Hubbewegung in Saug- und Druckrichtung des Pumpenkolbens ausführen kann.

Die als Ausführungsbeispiel in Fig. 2 gezeigte Kolbenpumpe 13 weist außerdem den Kolbenhub in ihren beiden Hubrichtungen erfassende Sensoren auf, die aus Näherungsschaltern 43 und 44 bestehen, mit deren Hilfe der Stellantrieb bzw. der Stellmotor 30 ein- und abgeschaltet sowie in seiner Drehrichtung umgekehrt werden kann. Die Näherungsschalter sind in einem Axialabstand zueinander am Gehäusekörper 27 in den Endbereichen seiner Schlitzöffnung 42 angeordnet und können von dem Stift beaufschlagt sein. Die Näherungsschalter 43 und 44 limitieren während der Ansaugphase der Meßprobe, während deren Vermessung im Meßzylinderraum 14 und beim Ausstoß der Meßprobe nach erfolgtem Meßvorgang aus dem Meßzylinderraum die Hubbewegungen des Pumpenkolbens 23 über dessen Stellantrieb. Solche Hubwegmeßeinrichtungen für die Meßkolben von Meßzylindern sind bei Meßeinrichtungen für die Messung der Gasbeladung von Flüssigkeiten vielfach bekannt, so daß sich insoweit eine weitere Beschreibung erübrigen kann.

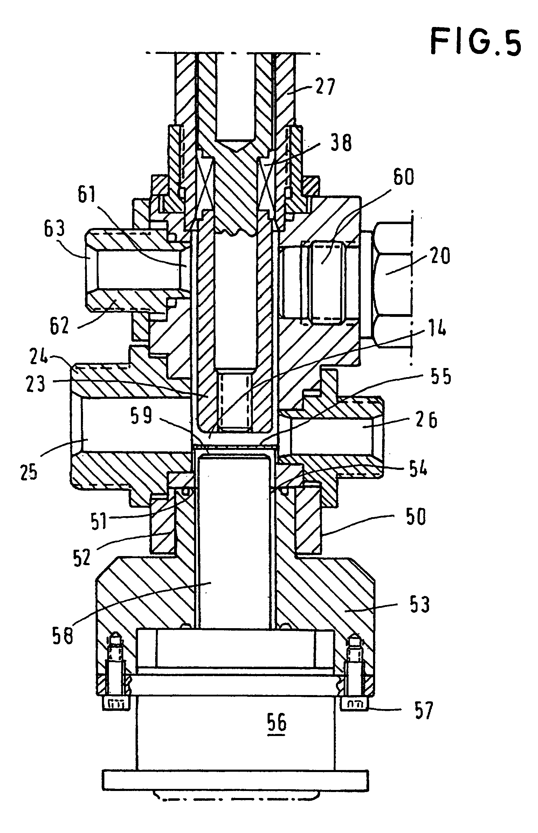

Fig. 5 zeigt den unteren, den Meßzylinderraum 14 aufweisenden Endabschnitt einer zweiten, besonders bevorzugten Ausführungsform der erfindungsgemäßen Kolbenpumpe. Diese zweite Ausführungsform entspricht in ihrem Aufbau hinsichtlich des nicht dargestellten, oberen Bereiches der Anordnung nach Fig. 2, so daß auf die detaillierte Beschreibung insoweit verzichtet werden kann.

Bei der zweiten, in Fig. 5 dargestellten Ausführungsform sind sowohl der Einlaß 25 als auch der Auslaß 26 für die Flüssigkeitsprobe als radial zum Zylinderraum 14 ausgerichtete Anschltisse ausgebildet, über die die Probemenge in den Meßzylinderraum 14 eingeleitet und nach erfolgter Messung wieder ausgestoßen wird. Am axialen (unteren) Ende 50 des Meßyzlinderraums 14 ist in dem Zylinderkörper 24 eine Axialbohrung 51 mit Innengewinde 52 vorgesehen, in die ein Halteflansch 53 eingeschraubt ist. Der Halteflansch 53 weist eine zentrale Bohrung auf, die zusammen mit der Bohrung 51 einen zylindrischen Durchlaß 54 zur Meßkammer 14 hin bildet, der an seinem vorderen, zum Pumpenkolben 23 hin weisenden Ende am Boden des Meßzylinderraums 14 von einer Membran 55 verschlossen sein kann.

Der Halteflansch 53 trägt einen Ultraschallerzeuger 56, der hierzu mit Befestigungsschrauben 57 am Flansch angeschraubt ist. Der Ultraschallerzeuger ist ein handelsübliches Bauteil und weist an seinem vorderen, zum Pumpenkolben hin weisenden Ende einen zylindrischen Ultraschallwellensender 58 auf, mit dem er in den axialen Durchlaß 54 gesteckt ist und mit seiner Stirnfläche 59 in Anlage mit der Membran 55 kommt.

Mit Hilfe des Ultraschallerzeugers kann die in dem Meßzylinderraum 14 aufgenommene Flüssigkeitsprobe mit Ultraschallwellen beschallt werden, wodurch der Exsorptionsvorgang, also das Trennen von Flüssigkeit und darin gelöstem Gas, gegenüber der in Fig. 2 dargestellten ersten Ausführungsform erheblich beschleunigt werden kann. Die von dem Ultraschallwellensender 58 über die Membran 55 in die Flüssigkeit eingeleiteten Ultraschallwellen bewirken also ein schnelleres Austreiben des Gases aus der Flüssigkeit, als dies allein durch den durch die Kolbenverstellung bewirkten Unterdruck möglich ist. Es hat sich gezeigt, daß die Zeit, während der der Unterdruck auf die Flüssigkeitsprobe zur Exsorption ausgeübt werden muß, um korrekte Meßergebnisse zu erhalten, um 50% und mehr verkürzt werden kann, wenn die Probe gleichzeitig mit Ultraschall beaufschlagt wird. Die Verkürzung dieser Meßzeit ist besonders vorteilhaft, da das Meßergebnis wesentlich zeitnäher zu dem Moment vorliegt, zu dem die Meßprobe tatsächlich aus dem Behälter entnommen wurde. Je kürzer die Zeit zwischen Entnahme der Meßprobe und Erhalt des Meßergebnisses ist, desto schneller und genauer kann der Gasgehalt in der Flüssigkeit auf den gewünschten Sollwert eingeregelt werden.

Bei dem in Fig. 5 dargestellten Ausführungsbeispiel der Kolbenpumpe ist oberhalb des Zulaufes 25 im Zylinderkörper 24 eine Anschlußöffnung 60 für den Temperatur- und Drucksensor 20 erkennbar, die sich radial gegenüber einer im Zylinderkörper 24 ebenfalls angeordneten Kontroll- und Serviceöffnung 61 befindet. Im Betrieb ist diese Kontroll- und Serviceöffnung mittels eines eingeschraubten Okulars 62 luftdicht verschlossen, wobei das Verhalten der gemessenen Flüssigkeit im Meßzylinderraum 14 durch das im Okular eingesetzte Schauglas 63 während der Messung beobachtet werden kann. Für Servicezwecke, beispielsweise zum Reinigen, kann das Okular 62 aus der Öffnung 61 herausgeschraubt werden, um dann einen Schlauch für Spülflüssigkeit od.dgl. anzuschließen.

Es ist erkennbar, daß das im Zusammenhang mit den Fig. 2 bis 5 beschriebene, im wesentlichen aus der Kolbenpumpe bestehende Meßgerät sehr kompakt baut, und daß über das als Spindelgetriebe ausgebildete Übertragungsgetriebe sehr exakte Hubbewegungen des den Meßkolben bildenden Pumpenkolbens 23 in beiden Hubrichtungen möglich sind, so daß über die von der Meßvorrichtung 20 gemessenen Meßwerte und die durch die Hubbewegungen des Pumpenkolbens 23 bewirkten Volumenveränderungen des Meßzylinderraums 14 eine sehr genaue Messung der Gasbeladung der im Meßzylinderraum 14 während des Meßvorgangs isolierten Probenmenge möglich ist. Dabei kann die Probemenge auch bei mehr als zwei unterschiedlichen Hubpositionen des Pumpenkolbens 23 im Meßzylinderraum 14 vermessen werden.

Über die physikalischen Größen "Überdruck und Unterdruck der Meßprobe" und über die hierbei ermittelten Druck- und Temperaturmeßwerte sowie die durch die Hubwege des Pumpenkolbens bedingten Volumenänderungen im Meßzylinderraum lassen sich Flüssigkeiten beliebiger Viskositäten unter Zugrundelegung des Gasgesetzes auf die in ihnen gelösten und freien Gase wirksam vermessen, wobei die ermittelten Meßwerte zur Einstellung der Gasbeladung der Flüssigkeiten genutzt werden können, wie dies beispielsweise für die in Fig. 1 gezeigte Anlage der Fall ist.

## Patentansprüche

1. Meßeinrichtung zur Messung der Gasbeladung einer flüssigen Kunststoffkomponente, mit einer in einem Umlaufkreis für die Probemenge angeordreten Kolbenpumpe, deren Pumpenkolben im Meßzylinderraum mittels eines Stellantriebs in beiden Hubrichtungen weggesteuert verstellbar ist, mit einer schaltbaren, ein Füll- und Ausstoßventil bildenden Ventilvorrichtung zur Befüllung des Meßzylinderraumes mit der zu vermessenden Probemenge und zur Ableitung der Probemenge aus dem Meßzylinderraum nach erfolgtem Meßvorgang, und mit einer dem Meßzylinderraum zugeordneten Druckmeßvorrichtung, mit der der Druck der im Meßzylinderraum bei geschlossener Ventilvorrichtung isolierten Probemenge bei verschiedenen vorgegebenen Hubpositionen des Pumpenkolbens zur Bestimmung der Gasbeladung meßbar ist, **dadurch gekennzeichnet, daß** der Stellantrieb (22) der Kolbenpumpe (13) aus einem Stellmotor (30) nebst Übertragungsgetriebe besteht und die Kolbenpumpe zugleich als eine die Meßprobe nach erfolgtem Meßvorgang aus dem Meßzylinderraum (14) in das System zurückführende Förderpumpe ausgebildet ist.

2. Meßeinrichtung nach Anspruch 1, **dadurch gekennzeichnet, daß** das Füllventil (16) und das Ausstoßventil (18) der Ventilvorrichtung aus unabhängig voneinander von der Schließstellung in die Öffnungsstellung und umgekehrt schaltbaren Ventilen bestehen.

3. Meßeinrichtung nach Anspruch 1 oder 2, **dadurch gekennzeichnet, daß** der Stellmotor (30) aus einem in seiner Drehrichtung reversierbaren, elektrischen Drehmotor besteht.

4. Meßeinrichtung nach einem der Ansprüche 1 bis 3, **dadurch gekennzeichnet, daß** das Übertragungsgetriebe aus einem Spindelgetriebe besteht, mit dessen gegen Drehung gesicherter verstellbarer Spindelmutter (36) der Pumpenkolben (23) verbunden ist.

5. Meßeinrichtung nach einem der Ansprüche 1 bis 4, **dadurch gekennzeichnet, daß** die Kolbenpumpe (13) aus einem den Meßzylinderraum (14) aufweisenden Zylinderkörper (24) besteht, der über einen das Übertragungsgetriebe bzw. das Spindelgetriebe aufnehmenden Gehäusekörper (27) mit einem Anschlußkörper (29) für den Anbau des Stellmotors (30) verbunden ist.

6. Meßeinrichtung nach Anspruch 5, **dadurch gekennzeichnet, daß** der Gehäusekörper (27) mit dem Zylinderkörper (24) und dem Anschlußkörper (29) lösbar verbunden ist.

7. Meßeinrichtung nach Anspruch 5 oder 6, **dadurch gekennzeichnet, daß** im Anschlußkörper (29) eine, Kupplungsvorrichtung (32) für die Kupplung des Stellmotors (30) mit dem Übertragungsgetriebe bzw. dem es bildenden Spindelgetriebe angeordnet ist.

8. Meßeinrichtung nach einem der Ansprüche 4 bis 7, **dadurch gekennzeichnet, daß** der Pumpenkolben (23) antriebsseitig einen Hohlkolbenansatz (39) aufweist, der die Hubspindel (33) mit der Spindelmutter (36) aufnimmt.

9. Meßeinrichtung nach Anspruch 8, **dadurch gekennzeichnet, daß** die Drehsicherung für die Spindelmutter (36) und den Pumpenkolben (23) aus einem am Hohlkolbenansatz (39) radial befestigten Stift (41) besteht, der sich in einem Axialschlitz (42) des Gehäusekörpers (27) führt.

10. Meßeinrichtung nach einem der Ansprüche 1 bis 9, **dadurch gekennzeichnet, daß** für den Kolbenhub in den beiden Hubrichtungen ermittelnde Sensoren vorgesehen sind.

11. Meßeinrichtung nach Anspruch 10, **dadurch gekennzeichnet, daß** die Sensoren aus den Stellantrieb steuernden Näherungsschaltern (43, 44) bestehen.

12. Meßeinrichtung nach einem der Ansprüche 1 bis 12, **dadurch gekennzeichnet, daß** der Meßzylinderraum (14) eingangsseitig der Kolbenpumpe (13) über das Füllventil (16) an einen die gasbeladene Flüssigkeit unter einem Vordruck enthaltenden, mit einer Gaszuführung versehenen Vorratsbehälter (1) und ausgangsseitig über das Ausstoßventil (18) an den Rücklauf (17) zum Vorratsbehälter (1) angeschlossen ist.

13. Meßeinrichtung nach Anspruch 12, **dadurch gekennzeichnet, daß** der Umlaufkreis für die Probenmessung gegenüber dem aus dem Vorratsbehälter (1) mit der gasbeladenen Flüssigkeit versorgten System über ein Absperrventil (12) absperrbar ist.

14. Meßeinrichtung nach einem der Ansprüche 1 bis 13, **dadurch gekennzeichnet, daß** eine Auswerte- und Steuereinheit (10) vorgesehen ist, die über elektrische Signal- und Steuerleitungen mit der Meßvorrichtung (20) und dem Stellantrieb (22) der Kolbenpumpe (13) sowie einem Gaszuführungsventil (6) verbunden ist, das in einer zu dem Vorratsbehälter (1) führenden Gaszuführungsleitung (5) angeordnet ist.

15. Meßeinrichtung nach dem Oberbegriff des Anspruchs 1 oder einem der Ansprüche 1 bis 14, **dadurch gekennzeichnet, daß** die der Kolbenpumpe (13) zugeordnete Meßvorrichtung (20) aus einer kombinierten Druck- und Temperaturmeßvorrichtung besteht.

16. Meßeinrichtung nach dem Oberbegriff des Anspruchs 1 oder einem der Ansprüche 1 bis 15, **gekennzeichnet durch** einen dem Meßzylinderraum (14) zugeordneten Ultraschall-erzeuger (56).

17. Meßeinrichtung nach einem der Ansprüche 1 bis 16, **dadurch gekennzeichnet, daß** der Einlaß (25) und der Auslaß (26) für die Befüllung und Entleerung radial seitlich in den Meßzylinderraum (14) münden.

18. Meßeinrichtung nach Anspruch 16 oder 17, **dadurch gekennzeichnet, daß** der Ultraschallerzeuger (56) an einem axialen Ende (50) des Meßzylinderraumes (14) angeordnet ist.

19. Meßeinrichtung nach einem der Ansprüche 16 bis 18, **dadurch gekennzeichnet, daß** der Ultraschallerzeuger (56) auswechselbar in einer Öffnung (54) am Meßzylinderraum (14) aufgenommen ist.

20. Meßeinrichtung nach Anspruch 19, **dadurch gekennzeichnet, daß** die Öffnung (54) von einer Membran (55) verschlossen ist.

21. Meßeinrichtung nach einem der Ansprüche 16 bis 20, **dadurch gekennzeichnet, daß** der Ultraschallerzeuger (56) in einem Halteflansch (53) aufgenommen und an diesem mittels Befestigungsschrauben (57) gesichert ist.

22. Meßeinrichtung nach einem der Ansprüche 1 bis 21, **dadurch gekennzeichnet, daß** der Meßzylinderraum (14) an seiner Zylinderwand bzw. dem Zylinderkörper (24) mindestens eine Anschlußöffnung (60) für einen Druck- und/oder Temperaturfühler (20) aufweist.

23. Meßeinrichtung nach einem der Ansprüche 1 bis 22, **dadurch gekennzeichnet**, **d a ß** in der Zylinderwand (24) zum Meßzylinderraum (14) eine verschließbare Kontroll- und/oder Serviceöffnung (61) vorgesehen ist.

24. Verfahren zur Bestimmung der Gasbeladung einer Flüssigkeit, insbesondere einer flüssigen Kunststoffkomponente, bei dem eine Probemenge aus einem Flüssigkeitsumlauf entnommen und in einen Meßzylinderraum einer als Kolbenpumpe ausgebildeten Meßeinrichtung eingebracht wird, worauf nach Verschließen der Füll- und Ausstoßventile der Kolbenpumpe der Initialdruck und/oder die Initialtemperatur der Probemenge gemessen wird und anschließend durch Verfahren des Pumpenkolbens ein Meßdruck im Meßzylinderraum erzeugt und das in der Probemenge enthaltene Gas exsorbiert wird, wobei zur Beschleunigung der Gasexsorption die Flüssigkeitsprobemenge mit Ultraschall beschallt wird und nach Exsorption des Gases die Gasmenge nach erneuter Messung des Druckes und/oder der Temperatur im Meßzylinderraum durch Bestimmung der Differenz zum Initialdruck bzw. der Initialtemperatur über das Gasgesetz ermittelt wird, und anschließend die Probemenge nach Öffnen des Ausstoßventils wieder in den Flüssigkeitsumlauf ausgestoßen wird.

## Claims

1. A measuring apparatus for measuring the gas loading of a liquid plastics component, comprising a piston pump which is disposed in a circuit for the sample quantity and the pump piston of which can be adjusted, with displacement control and by means of an actuating drive, in both directions of travel in the measuring cylinder chamber, comprising a switchable valve device forming a filling and ejection valve for filling the measuring cylinder chamber with the sample quantity to be measured and for carrying away the sample quantity from the measuring cylinder chamber after the measuring operation has been performed, and comprising a pressure measuring device which is associated with the measuring cylinder chamber and with which the pressure of the sample quantity, which is isolated in the measuring cylinder chamber when the valve device is closed, can be measured at different predetermined positions of displacement of the pump piston in order to determine the gas loading, **characterised in that** the actuating drive (22) of the piston pump (13) consists of a servomotor (30) together with a transmission gear and the piston pump is at the same time constructed as a feed pump which returns the sample quantity from the measuring cylinder chamber (14) to the system after the measuring operation has been performed.

2. A measuring apparatus according to claim 1, **characterised in that** the filling valve (16) and the ejection valve (18) of the valve device consist of valves which can be switched independently of each other from their closed position into their open position and vice versa.

3. A measuring apparatus according to claims 1 or 2, **characterised in that** the servomotor (30) consists of an electric torque motor, the direction of rotation of which can be reversed.

4. A measuring apparatus according to any one of claims 1 to 3, **characterised in that** the transmission gear consists of a spindle gear, to the spindle nut (36) of which, which is secured against rotation, the pump piston (23) is attached.

5. A measuring apparatus according to any one of claims 1 to 4, **characterised in that** the piston pump (13) consists of a cylinder body (24) which comprises the measuring cylinder chamber (14) and which is attached, via a housing body (27) which receives the transmission gear or the spindle gear, to a connection body (29) for the attachment of the servomotor (30).

6. A measuring apparatus according to claim 5, **characterised in that** the housing body (27) is detachably connected to the cylinder body (24) and the connection body (29).

7. A measuring apparatus according to claims 5 or 6, **characterised in that** a coupling device (32), for coupling the servomotor (30) to the transmission gear or to the spindle gear which forms the latter, is disposed in the connection body (29).

8. A measuring apparatus according to any one of claims 4 to 7, **characterised in that** the pump piston (23) has a hollow piston attachment (39) on its drive side which receives the displacement spindle (33) with the spindle nut (36).

9. A measuring apparatus according to claim 8, **characterised in that** the rotational locking for the spindle nut (36) and the pump piston (23) consists of a pin (41) which is fixed radially to the hollow piston attachment (39) and which is led into an axial slot (42) in the housing body (27).

10. A measuring apparatus according to any one of claims 1 to 9, **characterised in that** sensors are provided which determine the piston travel in both directions of travel.

11. A measuring apparatus according to claim 10, **characterised in that** the sensors consist of proximity switches (43, 44) which control the actuating drive.

12. A measuring apparatus according to any one of claims 1 to 12, **characterised in that** on the inlet side of the piston pump (13) the measuring cylinder chamber (14) is connected via the filling valve (16) to a supply vessel (1) which contains the gas-laden liquid under an initial pressure and which is provided with a gas supply, and on the outlet side it is connected via the ejection valve (18) to the return line (17) to the supply vessel (1).

13. A measuring apparatus according to claim 12, **characterised in that** the circuit for sample measurement can be shut off via an isolating valve (12) from the system which is supplied with the gas-laden liquid from the supply vessel (1).

14. A measuring apparatus according to any one of claims 1 to 13, **characterised in that** an evaluation and control unit (10) is provided which is connected via electrical signal and control lines to the measuring device(20) and to actuating drive (22) of the piston pump (13), and is also connected to a gas supply valve (6) which is disposed in a gas supply line (5) leading to the supply vessel (1).

15. A measuring apparatus according to the precharacterising clause of claim 1 or according to any one of claims 1 to 14, **characterised in that** the measuring device (20) which is associated with the piston pump (13) consists of a combined pressure and temperature measuring device.

16. A measuring apparatus according to the precharacterising clause of claim 1 or according to any one of claims 1 to 15, **characterised by** an ultrasonic generator (56) associated with the measuring cylinder chamber (14).

17. A measuring apparatus according to any one of claims 1 to 16, **characterised in that** the inlet (25) and the outlet (26) for filling and emptying lead radially into the measuring cylinder chamber (14) at the side thereof.

18. A measuring apparatus according to claims 16 or 17, **characterised in that** the ultrasonic generator (56) is disposed at an axial end (50) of the measuring cylinder chamber (14).

19. A measuring apparatus according to any one of claims 16 to 18, **characterised in that** the ultrasonic generator (56) is replaceably mounted in an opening (54) on the measuring cylinder chamber (14).

20. A measuring apparatus according to claim 19, **characterised in that** the opening (54) is closed by a membrane (55).

21. A measuring apparatus according to any one of claims 16 to 20, **characterised in that** the ultrasonic generator (56) is mounted in a holding flange (53) and is secured thereto by means of fastening screws (57).

22. A measuring apparatus according to any one of claims 1 to 21, **characterised in that** the measuring cylinder chamber (14) has at least one connection opening (60) for a pressure and/or temperature sensor (20) on its cylinder wall or on the cylinder body (24).

23. A measuring apparatus according to any one of claims 1 to 22, **characterised in that** a closable inspection and/or servicing opening (61) to the measuring cylinder chamber (14) is provided in the cylinder wall (24).

24. A method of determining the gas loading of a liquid plastics component, wherein a sample quantity is taken from a liquid circuit and is introduced into a measuring cylinder chamber of a measuring apparatus constructed as a piston pump, whereupon after closing the filling and ejection valves of the piston pump the initial pressure and/or the initial temperature of the sample quantity are measured and a measuring pressure is subsequently generated in the measuring cylinder chamber by the displacement of the pump piston and the gas contained in the sample quantity is desorbed, wherein in order to speed up the desorption of gas the sample quantity is acted upon by ultrasound and after the desorption of the gas the amount of gas is determined via the gas law, after a repeated measurement of the pressure and/or of the temperature in the measuring cylinder chamber, by determining the difference from the initial pressure or the initial temperature, and the sample quantity is subsequently ejected into the liquid circuit again after opening the ejection valve.

## Revendications

1. Dispositif destiné à mesurer la charge de gaz de composants liquides de matières plastiques, avec une pompe à piston agencée dans un circuit de circulation pour l'échantill et dont le piston peut être déplacé dans la chambre de mesure cylindrique au moyen d'un actionneur, par commande positive, et dans les deux sens de la course; avec un dispositif de soupape commutable, comportant une valve de remplissage et une soupape d'évacuation, pour le remplissage de la chambre de mesure cylindrique avec l'échantillon à mesurer et pour évacuer l'échantillon de la chambre de mesure cylindrique une fois le processus de mesure effectué; et avec un dispositif de mesure de là pression associé à la chambre de mesure cylindrique, avec lequel la pression de l'échantillon, isolé dans la chambre de mesure cylindrique, le dispositif de soupapes fermé, peut être mesurée pour déterminer la charge de gaz et ce, dans différentes positions de course prédéfinies du piston de la pompe; **caractérisé en ce que** l'actionneur (22) de la pompe à piston (13) est constitué d'un servomoteur (30) et d'un mécanisme de transmission, et **en ce que** la pompe à piston est conçue en même temps comme une pompe de transport retournant l'échantillon, une fois le processus de mesure effectué, de la chambre de mesure cylindrique (14) vers le système.

2. Dispositif de mesure selon la revendication 1, **caractérisé en ce que** la valve de remplissage (16) et la soupape d'évacuation (18) du dispositif de soupapes sont constituées de soupapes indépendantes l'une de l'autre, permettant une commutation entre les positions fermée et ouverte et inversement.

3. Dispositif de mesure selon la revendication 1 ou 2, **caractérisé en ce que** le servomoteur (30) est constitué d'un moteur électrique rotatif, dont le sens de rotation peut être inversé.

4. Dispositif de mesure selon l'une des revendications 1 à 3, **caractérisé en ce que** le mécanisme de transmission est constitué d'une commande à vérin, dont l'écrou (36), qui pouvant être déplacé est protégé contre toute rotation, est assemblé avéc le piston de la pompe (23).

5. Dispositif de mesure selon l'une des revendications 1 à 4, **caractérisé en ce que** la pompe à piston (13) est constituée d'un corps cylindrique (24) contenant la chambre de mesure cylindrique (14) et relié, via un carter accueillant le mécanisme de transmission ou la commande à vérin, à un corps de raccordement (29) destiné au montage du servomoteur (30).

6. Dispositif de mesure selon la revendication 5, caractêrisé en ce que le carter (27) est relié de manière amovible au corps cylindrique (24) et au corps de raccordement (29).

7. Dispositif de mesure selon la revendication 5 ou 6, **caractérisé en ce qu'**un dispositif de couplage (32), destiné à coupler le servomoteur (30) au mécanisme de transmission ou à la commande à vérin le constituant, est logé dans le corps de raccordement (29).

8. Dispositif de mesure selon l'une des revendications 4 à 7, **caractérisé en ce que** le piston de la pompe (23) présente, du côté du moteur, un fourreau (39) qui accueille le vérin (33) avec l'écrou de vérin (36).

9. Dispositif de mesure selon la revendication 8, **caractérisé en ce que** le dispositif anti-rotation pour l'écrou de vérin (36) et le piston de la pompe (23) est constitué d'une broche (41) qui est fixée sur un plan radial au niveau du fourreau (39) et s'adapte dans une fente axiale (42) du carter (27).

10. Dispositif de mesure selon l'une des revendications 1 à 9, **caractérisé en ce que** des capteurs sont prévus pour déterminer la course du piston dans les deux sens.

11. Dispositif de mesure selon la revendication 10, **caractérisé en ce que** les capteurs sont constitués de détecteurs de proximité (43, 44) guidant l'actionneur.

12. Dispositif de mesure selon l'une des revendications 1 à 12, **caractérisé en ce que** la chambre de mesure cylindrique (14) est reliée, du côté de l'entrée de la pompe à piston (13) et via la valve de remplissage (16), à un réservoir (1) contenant le liquide chargé de gaz et maintenu sous une pression amont et, du côté de la sortie et via la soupape d'évacuation (18), à la conduite de retour (17) vers le réservoir (1).

13. Dispositif de mesure selon la revendication 12, **caractérisé en ce que** le circuit de circulation destiné à mesurer l'échantillon peut être, via une vanne d'isolement (12), isolé du système alimenté en liquide chargé de gaz par le réservoir (1).

14. Dispositif de mesure selon l'une des revendications 1 à 13, **caractérisé en ce qu'**est prévu un module d'exploitation et de pilotage (10) qui est relié, via des câbles électriques de signalisation et de pilotage, au dispositif de mesure (20) et à l'actionneur (22) de la pompe à piston (13), ainsi qu'à une soupape d'admission du gaz (6), laquelle est logée dans une conduite d'amenée du gaz (5) conduisant au réservoir (1).

15. Dispositif de mesure selon la préambule de la revendication 1 ou selon l'une des revendications 1 à 14, **caractérisé en ce que** le dispositif de mesure (20) de la pompe à piston (13) est constitué d'un dispositif de mesure combinée de la pression et de la température.

16. Dispositif de mesure selon la préambule de la revendication 1 ou selon l'une des revendications 1 à 15, **caractérisé par** un générateur d'ultrasons (56) associé à la chambre de mesure cylindrique (14).

17. Dispositif de mesure selon l'une des revendications 1 à 16, **caractérisé en ce que** l'entrée (25) et la sortie (26) prévues pour remplir et vider le liquide débouchent latéralement, sur un plan radial, dans la chambre de mesure cylindrique (14).

18. Dispositif de mesure selon la revendication 16 ou 17, **caractérisé en ce que** le générateur d'ultrasons (56) est logé au niveau d'une extrémité axiale (50) de la chambre de mesure cylindrique (14).

19. Dispositif de mesure selon l'une des revendications 16 à 18, **caractérisé en ce que** le générateur d'ultrasons (56) est logé dans une ouverture (54) située au niveau de la chambre de mesure cylindrique (14), et peut être échangé.

20. Dispositif de mesure selon la revendication 19, **caractérisé en ce que** l'ouverture (54) est fermée par une membrane (55).

21. Dispositif de mesure selon l'une des revendications 16 à 20, **caractérisé en ce que** le générateur d'ultrasons (56) est retenu par une bride de maintien (53) et est maintenu dans celle-ci au moyen de vis de fixation (57).

22. Dispositif de mesure selon l'une des revendications 1 à 21, **caractérisé en ce que** la chambre de mesure cylindrique (14) présente, au niveau de sa paroi cylindrique ou du corps cylindrique (24), au moins une ouverture de raccordement (60) destinée à un capteur de pression et/ou de température (20).

23. Dispositif de mesure selon l'une des revendications 1 à 22, **caractérisé en ce qu'**une ouverture de contrôle et/ou de service (61) est prévue dans la paroi cylindrique (24) de la chambre de mesure cylindrique (14).

24. Procédé visant à déterminer la charge de gaz d'un composant liquide de matière plastique, suivant lequel un échantillon est prélevé d'un circuit de liquide et est introduit dans la chambre de mesure cylindrique d'un dispositif de mesure conçu comme une pompe à piston; après quoi la pression initiale et/ou la température initiale de l'échantillon est mesurée, une fois fermées la valve de remplissage et la soupape d'évacuation de la pompe à piston, puis le déplacement du piston de la pompe produit une pression de mesure dans la chambre de mesure cylindrique tandis que le gaz contenu dans l'échantillon est évacué; moyennant quoi l'échantillon de liquide est soumis à des ultrasons afin d'accélérer l'exsorpti du gaz, et la quantité de gaz, après exsorption du gaz et renouvellement de la mesure de la pression et/ou de la température à l'intérieur de la chambre de mesure cylindrique, est calculée selon la loi des gaz parfaits, en déterminant la différence avec la pression initiale ou la température initiale, l'échantillon étant ensuite expulsé de nouveau dans le circuit de liquide après ouverture de la soupape d'évacuation avec la pompe à piston.
